# EUROPEAN PATENT SPECIFICATION

(11) **EP 1 261 623 B1**
(45) Date of publication and mention of the grant of the patent: **04.11.2009**
(21) Application number: 02729592.2
(22) Date of filing: 10.01.2002
(51) Int. Cl.: C07K 5/00, C07K 5/06

(54) **NEW RETINOL DERIVATIVES, THE METHOD OF PREPARATIONS AND THE USES THEREOF**
RETINOL-DERIVATE, METHODE IHRER DARSTELLUNGEN UND IHRE VERWENDUNGEN
NOUVEAUX DERIVES DE RETINOL, PROCEDE DE PREPARATION ET D'UTILISATION DE CEUX-CI

(30) Priority: 11.01.2001 KR 2001001667; 09.01.2002 KR 2002001178
(43) Date of publication of application: 04.12.2002
(73) Proprietor: Chebigen Inc., Kangnam-gu, Seoul 135-080 (KR)
(72) Inventor: SIN, Hong-Sig, Kwanack-gu, Seoul 151-010 (KR); PARK, Si-Ho, Duckjin-gu, Jeonju 561-761 (KR); RHO, Young-Soy, Duckjin-gu, Jeonju 561-784 (KR); UM, Soo-Jong, Seongnam-city, Kyunggi-do 463-730 (KR); KWON, Youn-Ja, Seocho-gu, Seoul 137-070 (KR); PARK, Myoung-Soon, Eujeongbu-city, Kyunggi-do 480-070 (KR); HAN, Hye-Sook, Seoul 143-220 (KR); JUNG, Min-Sook, Wansan-gu, Jeonju 560-291 (KR); KIM CHO, So-Mi, Namyangju-city, Kyunggi-do 472-707 (KR); KIM, Dong-Myong, Yangchun-gu, Seoul 158-773 (KR); OH, Deok-Kun, Kwachun-city, Kyunggi-do 427-708 (KR); PARK, Jong-Sup, Kangnam-gu, Seoul 135-270 (KR)
(74) Representative: Dörries, Hans Ulrich
(86) International application number: PCT/KR2002/000041
(87) International publication number: WO 2002/055540

(56) References cited:
- WO-A-01/78676
- WO-A-95/16659
- WO-A-99/32105
- WO-A-99/52846
- CH-A- 393 310
- DE-A- 4 415 204
- US-A- 2 452 386
- US-A- 2 875 195
- US-A- 5 814 612
- US-A- 5 883 136
- US-A- 5 908 868
- HUMPHLETT W J ET AL: "SOLUBILIZATION OF CERTAIN ORGANIC COMPOUNDS BY USE OF ISOCYANATO ESTERS" JOURNAL OF ORGANIC CHEMISTRY, AMERICAN CHEMICAL SOCIETY. EASTON, US, vol. 26, July 1961 (1961-07), pages 2511-2515, XP001152524 ISSN: 0022-3263
- AZAIS-BRAESCO V ET AL: "RAPID SYNTHESIS AND PURIFICATION OF VITAMIN A ESTERS" JOURNAL OF THE AMERICAN OIL CHEMISTS' SOCIETY, AOCS PRESS, CHAMPAIGN, IL, US, vol. 69, no. 12, December 1992 (1992-12), pages 1272-1273, XP001152535 ISSN: 0003-021X
- MEUNIER: "Sur la constitution du pigment obtenu par chromatographie de l'axérophtol (vitamine A)" BULL. SOC. CHIM. BIOL., vol. 27, 1945, pages 186-190, XP001148048

## Description

### FIELD OF THE INVENTION

The present invention relates to novel retinol derivatives, the methods of their preparation and uses thereof.

Retinol derivatives are essential for the foetal development of the animal, for homeostatis, for vision, for morphogenesis, for skin aging and for the control of cell differentiation. Also, Retinol derivatives are considered to have utility as the agent for the inhibition or treatment of cancer caused by a virus or other factors due to the inhibition of reckless cell proliferation and the induction of cell differentiation or the induction of apoptosis.

Retinol derivatives maintain the activity of epithelial tissue and inhibit skin aging indirectly by interruption of the signal transmittance of the ultraviolet light Differentiation of stem cells to muscle neuron cells depends on the concentration of retinol. Therefore, retinol itself as well as its derivatives are widely used in various fields such as medicine, cosmetics, etc.

### BACKGROUND OF THE INVENTION

The methods of preparation of retinol via several stages are described in US 4035425, 4064183, 4092366. However, pure retinol prepared by the above methods is unstable in the light, easily photoisomerized and degraded with the consequence that its activity is affected and generally stabilizer is added into the commercial product of retinol.

Even though the methods of preparation of retinol derivatives bonded to various carbohydrates are disclosed in US 4473503 and 5631244 in order to overcome the stability problem cited above, the steps of the preparation are complex, non-economical and are not satisfactory in terms of the stability.

Accordingly, there is a need for retinol derivatives which are stable in the light and aqueous solution and the preparation method of which is simple and economical.

The present invention solves the above problems and the object of the present invention is to prepare novel retinol derivatives, the preparation methods of which having high yields, and uses thereof.

### DETAILED DESCRIPTION OF THE INVENTION

The present invention relates to novel retinol derivatives, methods of their preparation and uses thereof.

Carboester derivatives of retinol in the present invention comprise carboester linkage between retinol and a peptide having a functional group of COOH, wherein the retinol derivative has the formula I 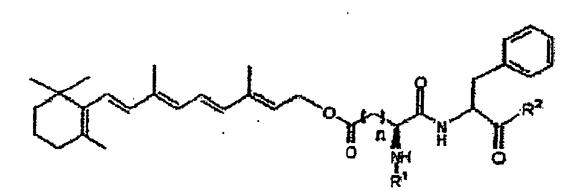 wherein either R¹ is H, CHO, retinoic acid (RA), Ac or Boc;
R² is OH OCH₃, OC₂H₅, retinol, or conjugated linoleic acid (CLA); and
n is an integer of 1-6,
or n is 1, R² is OCH₃, R¹ is 3,3-dimethyl-butyl or an N-protecting-group, and the phenylalanine is L-phenylalanine.

In the following, the present invention will be explained in more detaiL

According to the present invention, the structure of carboester derivatives of retinol is represented as follows. Among carboester derivatives of retinol of the present invention, representative examples having carboester linkages between peptides with COOH functional group and retinol correspond the following formula 1.

In the following formula 1, the peptide with COOH functional group is selected from a peptide comprising N-L-α-aspatyl-L-phenylalanine-1-methylester(APM; aspartame), N-protecting group-aspartame, neotam, etc.

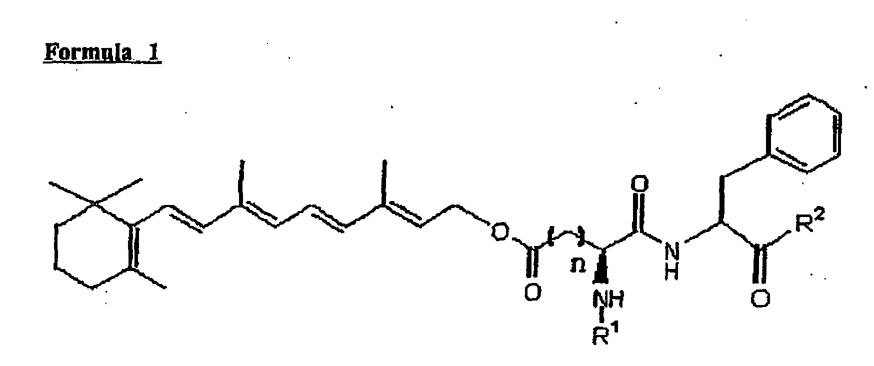

Further described herein is a structure of carboester derivatives of retinol having carboester linkage between amino acid with di-COOH functional group and retinol that corresponds to the following formula 2.
wherein either R¹ is H, CHO, retinoic acid (RA), Ac or Boc;
R² is OH, OCH₃, OC₂H₅, retinol, or conjugated linoleic acid (CLA); and
n is an integer of 1-6,
or n is 1, R² is OCH₃, R¹ is 3,3-dimethyl-butyl or an N-protecting-group, and the phenylalanine is L-phenylalanine.

In the following formula 2, the amino acid with di-COOH is selected from the group comprising aspartic acid, N-protecting group-aspartic acid, glutamic acid, N-protecting group-glutamic acid, α-asparty-L-phenylalanine (α-AP), and N-protecting group-α -aspartyl-L-phenylalanine. 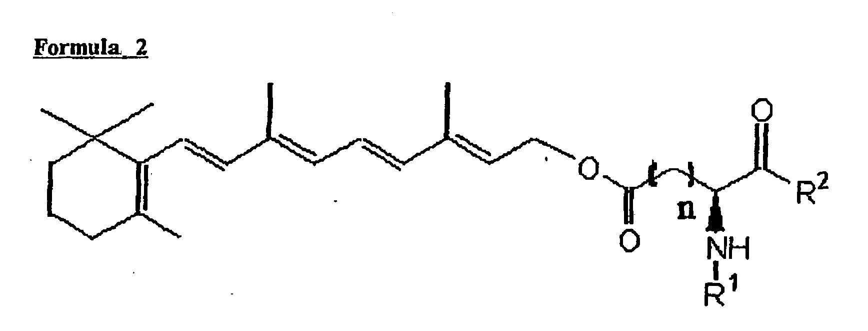 wherein, R¹ is H, CHO, Retinoic acid(RA), Ac or Boc, R² is OH, OCH₃, OC₂H₅, Retinol or CLA, and n is the integer of 1∼6.

Further described herein is a structure of carboester derivatives of retinol having a carboester linkage between the compounds with COOH functional group and multiple double bonds on the carbon chain and retinol that corresponds to the following formula 3.

In the following formula 3, the compounds with COOH functional group and multiple double bonds on the carbon chain are selected from the group comprising oleic acid, linolenic acid, prodlure, leukotrienes, etc instead of conjugated linoleic acid (CLA).

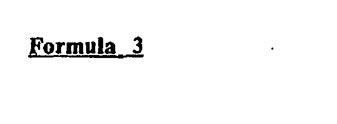

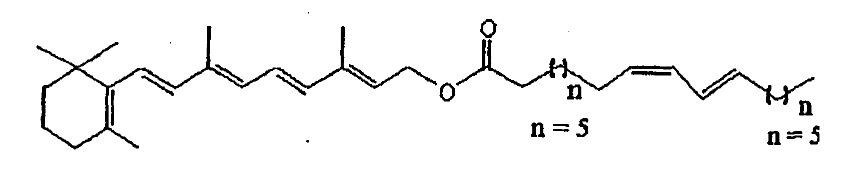

Further described herein is a structure of carboester derivatives of retinol having a carboester linkage between the compounds with di-COOH functional group and one double bond and retinol that corresponds to the following formula 4.

In the following formula 4, the compounds with di-COOH functional group and one double bond are selected from the group comprising maleic acid, fumaric acid, mesaconic acid, etc. 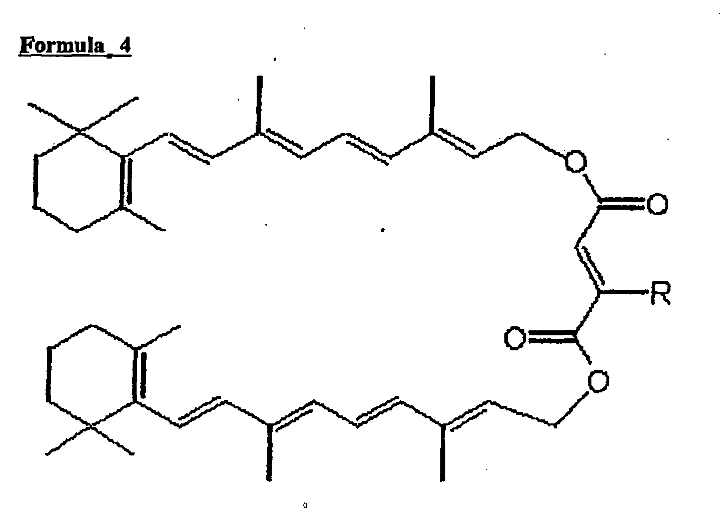 wherein, R is H, CH₃ or C₂H₅.

Further described herein is a structure of ether derivatives of retinol having an ether linkage between the compounds with OH functional group and retinol corresponds to the following formula 5.

In the following formula 5, the compounds with OH functional group are selected from the group comprising alkyl alcohol, aryl alcohol, dienyl alcohol, trienyl alcohol, etc instead of retinol.

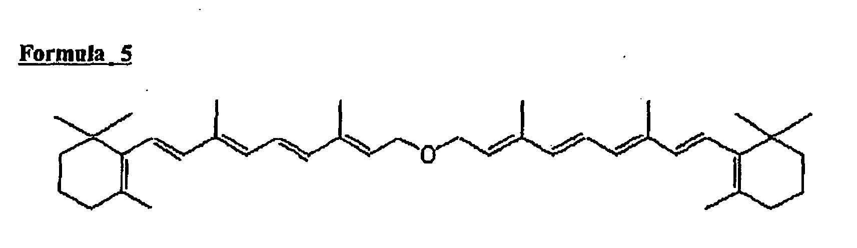

A method for preparing retinol carboester derivatives of the present invention consists of the following stages; 1) a converting stage of retinyl acetate to pure retinol by reacting retinyl acetate with an inorganic salt in the methanolic solvent and extracting retinol with ether, 2) a binding stage of a peptide having a functional group of the COOH, to retinol under the presence of a condensing agent and a catalyzing agent in the solvent, and 3) a separating, purifying and recovering stage of retinol carboester derivatives.

A method for preparing the retinol carboester derivatives of the present invention is explained by stages as follows:
At the first stage retinyl acetate, which is generally commercialized, is reacted with an inorganic salt in a methanolic solvent at 25-40°C in the dark room to prevent photoisomerization during the reaction, and the reaction mixture is extracted with ether as solvent. At this time, an inorganic salt used is 1-3 equivalents and the extracting solvents comprises ethers such as diethylether or tetrahydrofuran(THF). The ether is used as a solvent because the residual inorganic salt dissolved in the methanolic solvent reacts with the extracting solvents such as ethylacetate and converts to retinyl acetate.
At the second stage, a peptide having a functional group of COOH is reacted with a condensing agent and methylene chloride (or organic solvent) to activate the acidity of the chemical or to convert to acyl halide. Thereafter the retinol carboesters are prepared by adding the pure retinol in the reaction mixture. The condensing agent comprises N,N'-dicyclohexylcarbodiimide (DCC), 1-ethyl-3-(3-dimethylaminopropyl) carbodiimide hydrochloride (EDCI), N,N'-carbonyldiimidazole (CDI), N,N'-sulfryldiimidazole (SDI)), or SO₂Cl, and the catalyzing agent which catalyze the condensing reaction comprises N,N-dimethylaminopyridine (DMAP).

The third stage is separating the pure retinol carboester derivatives. The reaction mixture is column chromatographed on a special silicagel [Reverse-phase, Merck Silicagel 60 RP 18(40-63) µm].

Further described herein is a method for preparing the retinol ether derivatives consisting of 1) a converting stage of retinyl acetate to pure retinol, 2) a binding stage of the chemical containing the functional group of OH and 3) a separating, purifying and recovering stage of retinol ether derivatives.

A process for preparing the retinol ether derives as described herein is explained by stages as follows:
At the first stage, generally commercially available retinyl acetate is reacted with an inorganic salt in methanolic solvent at 25-40°C in the dark room to prevent photoisomerization and then the reaction mixture is extracted with a solvent such as ethers.
At the second stage, after the removal of the solvent, a chemical containing the functional group of OH, a natural or isolated and purified retinol, Diethylazodicarboxylate(DEAD) and Triphenylphosphate(Ph3P) are added in the methylene solvent, and the reaction mixture is reacted at room temperature to prepare retinol ether derivatives.
The third stage is a separating stage of the pure retinol derivatives. The reaction mixture is column chromatographed on a special silicagel [Reverse-phase, Merck Silicagel 60 RP 18(40-63) m].

Base, condensing agents and catalyzer are not limited to the aforementioned ingredients used in the method of preparing said retinol derivaties, and can extend to any of the standard ingredients generally known in the field of the invention provided that the ingredients do not harmfully influence the reaction.

Retinol derivatives according to the present invention can be utilized in medicines, cosmetics, soap, shampoo and functional food in order to prevent and improve skin aging resulting in wrinkle, coarse skin, dryness and abnormal keratinization.

Pharmaceutical compositions comprising retinol derivatives of the present invention can be utilized as agent for the treatment and prevention of skin cancer, pimple and wrinkle with age, pigmentation, coarse skin and flappy skin. For the purpose of this invention, the methods of administration are to include the oral, local, transdermal, intranasal, inhalation, ocular, rectal, intravenous, intraperitoneal, intramuscular, intraarterial, or subcutaneous route. A transdermal formulation such as a lotion, ointment, gel, cream, patch or spray is a desirable pharmaceutical formulation.

Even though the recommended daily administration dosage depends on the age, weight, and desease of the patient, a suggested dose is 0.01 to 80mg per kg body weight and 1 to 3 administrations per day.

Also, cosmetic compositions containing retinol derivatives of the present invention can be added into the cosmetics for the purpose of prevention and improvement of skin abnormality such as freekles or senile chromelasma. A cosmetic formulation such as a toning lotion, nourishing lotion, massage cream, nourishing cream, pack, gel or skin adhesive type is a desirable formulation but is not intended to limit in any way.

Also, food compositions containing retinol derivatives of the present invention can be added into various food, meat, chocolate, snack, cookies, pizza, instant noodles, ice creams, alcohol beverages, vitamins, or health food for the purpose of prevention and improvement of skin abnormality such as pimple, freckles, wrinkle, pigmentation, coarse skin and flappy skin.

### BRIEF DESCRIPTION OF THE FIGURES

Figure 1 shows the cell toxicity analysis of retinol-(N-formyl-aspartame) of this invention.
Figure 2 shows the activity control analysis of retinol-(N-formyl-aspartame) of this invention on retinoic acid receptor/retinoid X receptor (RAR/RXR).
Figure 3 shows the activity control analysis of retinol-mesaconic acid as described herein on retinoic acid receptor (RAR)
Figure 4 shows the activity control analysis of retinol-fumaric acid as described herein on retinoic acid receptor (RAR)
Figure 5 shows the activity control analysis of retinol-retinol as described herein on retinoic acid receptor (RAR)
Figure 6 shows the activity control analysis of retinol-(N-formyl-aspartame) of this invention on activation protein-1 (AP-1).

### Example

Practical and presently preferred embodiments of the present inventions are illustrative as shown in the following Examples. However, this invention is not limited to these examples.

### Example 1a : Preparation of (2E,4E,6E,8E)-3,7-dimethyl-9-(2,6,6-trimethyl-1-cyclohexenyl)-2,4,6,8-nonatetraeny(3S)-4-[(1-benzyl-2-methoxy-2-oxoethyl)amino]-3-formylamino-4-oxobutanoate

N-formyl aspartame (843.3mg), methylene chloride 10mℓ and a minimum amount of dimethylformamide (DMF) were added to nitrogen filled three mouth round bottom flask, and melted. The reaction mixture was cooled down to 0°C, 1-ethyl-3-(3-dimethylaminopropyl)carbodiimide hydrochloride [EDCI](683mg) was added slowly, and stirred for approximately thirty minutes Retinol was added to the reaction mixture, a small amount of N,N'-dimethylaminopyridine (DMAP) was added immediately, and stirred for approximately 1∼3 hours. After the reaction was finished, eluent was removed under low pressure, the residues were melted with ethyl acetate and washed several times with water and Brine. The organic phase was dried with anhydrous MgSO₄, concentrated under low pressure and separated with reverse-phase column chromatography.

yield: 82%

NMR data : 8.81(s, 1H), 7.12-7.13(t, 3H, J=7.08Hz), 7.04(d, 2H, J=7.81Hz), 6.64(dd, 1H, J=13.18Hz), 6.27(d, 1H, J=15.14Hz), 6.18(d, 1H, J=16.1Hz), 6.11(d, 1H, J=11.72Hz), 6.07(d, J=7.32Hz), 5.56(t, J=7.32Hz), 5.2(s, 1H), 4.75(d, J=7.32Hz), 4.12(t, J=7.32Hz), 3.70(s, 3H), 3.04(d, J=6.84Hz), 2.94(dd, 2H, J=4.39Hz), 2.65(d, 2H, J=6.84Hz), 2.01(t, 2H, J=5.86), 1.95(s, 3H), 1.88(s, 3H), 1.17(s, 3H), 1.62(m, 2H), 1.46(m, 2H), 1.02(s, 6H)

### Example 1b : another example of Example 1a

All procedure were done as Example 1a except for the utilization of N,N'-carbonyldiimidazole (CDI) as coupling reagent.

### Example 1c : another example of Example 1a

All procedures were done as Example 1a except for the utilisation of SO₂Cl as coupling reagent.

### Example 1d : another example of Example 1a

All procedure were done as Example 1a except for the utilization of N,N'-sulfuryldiimidazole (SDI) as coupling reagent.

### Example 2 : Preparation of (2E,4E,6E,8E)-3,7-dimethyl-9-(2,6,6-trimethyl-1-cyclohexenyl)-2,4,6,8-nonatetraenyl(3S)-4-[(1-benzyl-2-methoxy-2-oxoethyl)amino]-3-amino-4-oxobutanoate, hydrochloride

Aspartame hydrochloride (150mg), methylene chloride 10mℓ and a minimum amount of dimethylformamide (DMF) were added to nitrogen filled, three mouth-round bottom flask and melted. The reaction mixture was cooled down to 0°C, 1-ethyl-3-(3-dimethylaminopropyl)carbodiimide hydrochloride [EDCI](128mg) was added slowly, and stirred for approximately 30 minutes. Retinol (130mg) was added into the reaction mixture, a small amount of N,N'-dimethylaminopyridine (DMAP) was added immediately, and stirred for approximately 1∼3 hours. After the reaction was finished, eluent was removed under low pressure, the residues were melted with ethyl acetate and washed several times with water and Brine. The organic phase was dried with anhydrous MgSO₄, concentrated under low pressure and separated with column chromatography.

yield: 62%

NMR data; 7.11-7.12(t, 3H, J =7.08Hz), 7.0(d, 2H, J=7.81Hz), 6.61(dd, 1H, J=13.18Hz), 6.20(d, 1H, J=15.14Hz), 6.10(d, 1H, J=16.1Hz), 6.05(d, 1H, J=11.72Hz), 6.02(d, J=7.32Hz), 5.53(t, J=7.32Hz), 5.10(bs, 1H), 4.70(d, J=7.32Hz), 4.12(t, J=7.32 Hz), 3.70(s, 3H), 3.14(d, J=6.84Hz), 2.84(dd, 2H, J=4.39Hz), 2.45(d, 2H, J=6.84Hz), 2.01(t, 2H, J=5.86), 1.75(s, 3H), 1.68(s, 3H), 1.17(s, 3H), 1.52(m, 2H), 1.26(m, 2H), 1.02(s, 6H)

### Reference Example 3 :Preparation of Di[(2E,4E,6E,8E)-3,7-dimethyl-9-(2,6,6-trimethyl-1-cyclohexenyl)-2,4,6,8-nonatetraenyl](2S)-2-[(tert-butoxycarbonyl)amino] butanedioate

N-Boc aspartic acid (1.2mg), methylene chloride 20mℓ and a minimum amount of dimethylformamide (DMF) were added to nitrogen filled, three mouth- round bottom flask and melted. The reaction mixture was cooled down to 0°C, 1-ethyl-3-(3-dimethylaminopropyl)carbodiimide hydrochloride [EDCI](1.36g) was added slowly, and stirred for approximately 30 minutes. Retinol (0.51g) was added to the reaction mixture, a small amount of N,N'-dimethylaminopyridine (DMAP) was added immediately, and stirred for approximately 1∼3 hours. After the reaction was finished, eluent was removed under low pressure, the residues were melted with ethyl acetate and washed several times with water and Brine. The organic phase was dried with anhydrous MgSO₄, concentrated under low pressure and separated with column chromatography.

yield : 53%

NMR data : 6.64(dd, 3H, J=13.18Hz), 6.23(d, 2H, J=15.14Hz), 6.07(d, 2H, J=16.11Hz), 6.05(d, 2H, J=1.1.23Hz), 5.54(t, 2H, J=6.60Hz), 4.77(d, 2H, J=7.23Hz), 4.71(d, 2H, J=7.32Hz), 2.97(dd, 2H, J=4.39Hz), 2.01(t, 4H, J=5.86Hz), 1.95(s, 6H), 1.88(s, 6H), 1.17(s, 6H), 1.62(m, 4H), 1.46(m, 4H), 1.42(s, 9H), 1.02(s, 12H)

### Reference Example 4 : Preparation of Di[(2E,4E,6E,8E)-3,7-dimethyl-9-(2,6,6-trimethyl-1-cyclohexenyl)-2,4,6,8-nonatetraenyl](2S)-2-aminobutanedioate

Aspartic acid (160mg), N,N'-dicyclohexylcarbodiimide (DCC) (720mg), methylene chloride 10mℓ and minimum amount of dimethylformamide (DMF) were added to nitrogen filled, three mouth- round bottom flask and melted. A small amount of N,N'-dimethylaminopyridine (DMAP) was added. Retinol (324mg) was added into the reaction mixture, and stirred for approximately 1∼3 hours. After the reaction was finished, eluent was removed under low pressure, the residues were melted with ethyl acetate and washed several times with water and Brine. The organic phase was dried with anhydrous MgSO₄, concentrated under low pressure and separated with column chromatography.

yield : 50%

NMR data : 6.54(dd, 3H, J=13.18Hz), 6.08(d, 2H, J=15.14Hz), 6.07(d, 2H, J=16.11Hz), 6.05(d, 2H, J=11.23Hz), 5.54(t, 2H, J=6.60Hz), 4.57(d, 2H, J=7.23Hz), 4.51(d, 2H, J=7.32Hz), 2.67(dd, 2H, J=4.39Hz), 2.01(t, 4H, J=5.86Hz), 1.95(s, 6H), 1.88(s, 6H), 1.27(s, 6H), 1.62(m, 4H), 1.46(m, 4H), 1.02(s,12H)

### Reference Example 5 Preparation of Di[(2E,4E,6E,8E)-3.7-dimethyl-9-(2,6,6-trimethyl-1-cyclohexeny)-2,4,6,8-nonatetraenyl] 2-(acetylamino)succinate

N-acetyl aspartic acid (610mg), N,N'-dicyciohexylcarbodiimide (DCC) (720mg), methylene chloride 10ml and a mminimum amount of dimethylformamide (DMF) were added to nitrogen filled, three mouth- round bottom flask and melted. A small amount of N,N'-dimethylaminopyridine (DMAP) was added. Retinol (324 mg) was added into the reaction mixture, and stirred for approximately 1∼3 hours. After the reaction was finished, eluent was removed under low pressure, the residues were melted with ethyl acetate and washed several times with water and Brine (20mlx2). The organic phase was dried with anhydrous MgSO₄, concentrated under low pressure and separated with column chromatography.

yield: 50%

NMR data : 6.54(dd, 3H, J=13.18Hz), 6.08(d, 2H, J=15.14Hz), 6.07(d, 2H, J=16.11Hz), 6.05(d, 2H, J=11.23Hz), 5.54(t, 2H, J=6.60Hz), 4.57(d, 2H, J=7.23Hz), 4.51(d, 2H, J=7.32Hz), 2.67(dd, 2H, J=4.39Hz), 2.01(t, 4H, J=5.86Hz), 1.95(s, 6H), 1.88(s, 6H), 1.17(s, 6H),1.62(m, 4H),1.46(m, 4H). 1.42(s, 9H), 1.02(s, 12H)

### Reference Example 6 : Preparation of (2E,4E,6E,8E)-3,7-dimethyl-9-(2,6,6-trimethyl-1-cyclohexenyl)-2.4.6,8-nonatetraenyl (9Z,11E)-9,11-octadecadienoate

1-ethyl-3-(3-dimethylaminopropyl) carbodiimide hydrochloride [EDCI] (0.71g, 3.67mmol) and anhydrous methylene chloride 15mℓ were added to nitrogen filled, three mouth- round bottom flask and melted The reaction mixture was cooled down to 0°C. Conjugated linoleic acid was added, and stirred for approximately 30 minutes. Retinol (0.87g, 3.05mmol) and N,N'-dimethylaminopyridine (DMAP) solubilized in anhydrous methylene chloride 7ml were added to the reaction mixture, and stirred for 4 hours at room temperature. The organic phase was washed with water (20mlx2) and brine (20mlx2) and dried with anhydrous MgSO₄, concentrated under low pressure and separated with column chromatography (solvent: ethylacetate/hexane=1/8).

yield : 80%

NMR data(400MHz. CDCl₃) : 6.64(dd, 1H, J=15.14Hz, 11.23Hz), 6.29(d, 1H, J=15.14Hz), 6.17(d, 1H, J=16.11Hz), 6.12(d, 1H, J=16.11 Hz), 6.09(d, 1H, 1=11.23 Hz), 5-96(m, 1H, J=10.74Hz, 5.86Hz), 5.93(m, 1H, J=10.74Hz), 5.61(m, 1H, J=6.83Hz, 7.32Hz), 532(t, 1H, J=7.32Hz), 5.30(m, 1H, J=10.74Hz, 6.83Hz), 4.72(d, 2H, J=7.32 Hz), 2.30(t, 2H, J=5.86Hz), 2.14(m, 2H, J=5.86Hz, 10.74Hz), 2.08(m, 2H, J=5.86Hx, 7.32Hz), 2.01(m, 2H, J=5.86Hz), 1.96(s, 3H), 1.89(s, 3H), 1.71(s, 3H), 1.60(m, 2H, J=5.86Hz), 1.47(m, 2H, J=5.86Hz), 1.29(m, 16H,J=5.86Hz), 1.02(s, 16H), 0.87(t, 3H, J=7.32Hz)

### Reference Example 7: Preparation of Di[(2E,4E,6E,8E)-3,7-dimethyl-9-(2,6,6-trimethyl-1-cyclohexenyl)-2,4,6,8-nonatetraenyl]malate

1-ethyl-3-(3-dimethylaminopropyl)carbodiimide hydrochloride [EDCI] (334.6mag, 1.75mmol) and anhydrous methylene chloride 5mℓ were added to nitrogen filled, three mouth- round bottom flask and melted. The reaction mixture was cooled down to 0°C. Maleic acid (78mg, 0.58mmol) was added, and stirred for approximately 3.0 minutes. Retinol (500mg, 1.75mmol) and N,N'-dimethylaminopyridine solubilized in anhydrous methylene chloride 6ml were added into the reaction mixture, and stirred for 4 hours at room temperature. The organic phase was washed with water (20mlx2) and brine (20mlx2) and dried with anhydrous MgSO₄, concentrated under low pressure and separated with column chromatography (solvent: ethylacetate/hexane=1/30).

yield: 48%

NMR data(400MHz, CDCl₃) : 6.60(dd, 2H, J=15.14Hz, 11.23Hz) 6.23 (d, 2H, J=15.14Hz), 6.17(d, 2H, J=16.11Hz), 6.13(d, 2H, J=16.11Hz). 6.07(d, 2H, J=11.23Hz), 5.50(t; 2H, J=7.32Hz), 4.73(d, 2H, J=7.32Hz), 4.65(d, 2H, J=7.32Hz), 5.23(bq, 1H, J=16.60Hz), 3.00(d, 1H, J=16.60Hz), 2.90(d, 1H, J=16.60Hz), 1.98(m,4H, J=5.86Hz), 1.94(s, 6H), 1.83(s, 6H), 1.68(s, 6H), 1.61(m, 4H, J=5.86Hz), 1.46(m, 4H, J=5.86Hz), 1.02(s, 12H)

### Reference Example 8 : Preparation of Di[(2E, 4E, 6E, 8E)-3,-7-dimethyl-9-(2, 6, 6-trimethyl-1-cyclohexenyl)-2,4,6,8-nonatetraenyl] (E)-2-butenedioate

1-ethyl-3-(3-dimethylaminopropyl) carbodiimide hydrochloride [EDCI] (1.4g, 7.32mmol) and anhydrous methylene chloride 20mℓ were added to nitrogen filled, three mouth- round bottom flask and melted. The reaction mixture was cooled down to 0°C. Fumaric acid (0.39g, 3.05mmol) was added, and stirred for approximately 30 minutes. Retinol (1.75g, 6.10mmol) and N,N'-dimethylaminopyridine solubilized in anhydrous methylene chloride 7ml were added into the reaction mixture, arid stirred for 4 hours at room temperature. The organic phase was washed with water (20mlx2) and brine (20mlx2) and dried with anhydrous MgSO₄, concentrated under low pressure and separated with column chromatography (solvent: ethylacetate/hexane=1/30).

yield: 50%

NMR data: 6.86(s, 2H), 6.65(dd, 2H, J=24Hz), 6.08∼6.29(m, 8H), 5.63(t, 2H, J=15Hz), 4.85(d, 4H, J=15Hz), 1.99∼2.03(m, 4H), 1.96(s, 6H), 1.91(s, 6H), 1.70(s, 6H), 1.59∼1.62(m, 4H), 1.45∼1.47(m, 4H), 1.02(s, 12H)

### Reference Example 9 :Preparation of Di[(2E,4E,6E,8E)-3,7-dimethyl-9-(2,6,6-trimethyl-1-cyclohexenyl)-2,4,6,8-nonatehaenyl] (E)-2-methyl-2-butenedioate

1-ethyl-3-(3-dimethylaminopropyl) carbodiimide hydrochloride [EDCI] (I.4g, 7.32mmol) and anhydrous methylene chloride 20mℓ were added to nitrogen filled, three mouth- round bottom flask and melted. The reaction mixture was cooled down to 0°C. Mesaconic acid (0.39g, 3.05mmol) was added, and stirred for approximately 30 minutes. Retinol (1.75g, 6.10mmol) and N,N'-dimethylaminopyridine solubilized in anhydrous methylene chloride 7ml were added to the reaction mixture, and stirred for 4 hours at room temperature. The organic phase was washed with water (20mlx2) and brine (20mlx2) and dried with anhydrous MgSO₄, concentrated under low pressure and separated with column chromatography (solvent: ethylacetate/hexane=1/30).

yield : 53%

NMR data: 6.79(s,1H), 6.65(dd,2H, J=27Hz), 6.08∼6.29(m, 8H), 5.63(t, 2H, J=14Hz), 4.83(dd, 4H, J=16Hz), 2.29(s, 3H, J=-4.39Hz), 1.99∼2.03(m, 4H), 1.96(s, 6H), 1.91(s, 6H), 1.67(s, 6H), 1.58∼1.62(m, 4H), 1.45∼1.47(m, 4H), 1.02(s, 12H)

### Reference Example 10 : Preparation of (1B,3E,5E,7E)-9[(2E,4E,6E,8E)-3,7-dimethyl-9-(2,6,6-trimethyl-1-cyclohexenyl)-2,4,6,8-nonatetraenyl]oxy-3,7-dimethyl-1-1(2,6,6-trimethyl-1-cyclohexenyl)-1,3,5,7-nonateraene

Retinol (0.6g, 2.10mmol), DEAD (Diethylazodicarboxylate) (0.3g, 1.15mmol), Ph₃P (0.33g, 1.15mmol) and anhydrous methylene chloride 20mℓ were added to nitrogen filled, three mouth- round bottom flask, melted and vigorously stirred for approximately 20 minutes. After the reaction was finished, 50ml of water were added into the reaction mixture and ethylacetate (30mlx2) was used for extraction. The organic phase was washed with water (50ml) and brine (50ml) and concentrated under low pressure and separated with column chromatography (solvent: ethylacetate/hexane=1/1).

yield: 84%

NMR data : 6.05(q, 4H, J=16.11Hz), 5.14(br s, 6H), 4.16(q, 4H, J=7.32Hz), 1.99(s, 6H), 1.74(s, 6H), 1.68(s, 6H), 1.63(t, 4H, J= 5.86Hz), 1.45(m, 4H), 1.01(s, 6H), 1.00(s, 6H)

### Experimental Example 1 : Cell toxicity test of retinol-(N-formyl aspartame)

The cell lines utilized for the cell toxicity test of retinol derivatives were SK-Hep-1 (liver cancer), MDA-MB-231 (breast cancer), HaCAT (skin cancer), and HCT116 (colon cancer). Those cell lines were maintained and cultured in DMEM (Dulbecco's Modified Eagle's Medium)/10% FBS containing 10% fetal bovine serum (FBS) (GibcoBRL, Gaithersburg, MD). 3 × 10³ cells, 100µℓ media per well were distributed into 96 well microtiter plates and cultured for 24 hours. Each cultured cell line was treated with various concentrations of retinol derivatives (0, 500, 1000, 5000 nM) for 4 days and living cells were counted with a hemocytometer to measure the inhibition effect of cell proliferation.

The retinol derivatives utilized for the experiments were retinol-(N-formyl-aspartame, retinol, and 4-HPR[N-(4-hydroxyphenyl)retineamide] (Sigma Co., St. Louis, MO). Those derivatives were eluted in dimethylsulfoxide and the concentration of dimethylsulfoxide added into culture media was kept at not over 0.01 %.

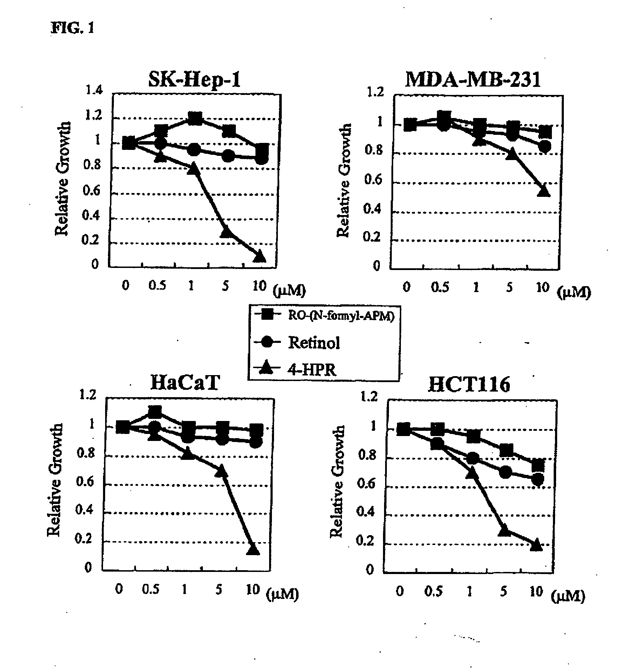

As shown in Figure 1, the result of treatment with different concentrations of the retinol derivatives were as follows. Retinol-(N-formyl-aspartame) shows no toxicity on the SK-Hep-1 (liver cancer), MDA-MB-231 (breast cancer), and HaCAT (skin cancer) cell line, and show less toxicity than retinol on HCT116 (colon cancer) cell line.

### Experimental Example 2 : Activity test of retinoic acid receptor/retinoid X receptor (RAR/RXR) using retinol-(N-formyl aspartame)

To analyze the effects of retinol-(N-formyl aspartame) on the activity of retinoic acid receptor, skin cancer (HaCAT) cell lines were utilized to measure the activity of retinoic acid receptor/retinoid X receptor.

Recombinant DNA, DR5-tk-CAT or DR1-tk-CAT consisting of DR 1 or DR5, effectors of retinoic acid receptor/ retinoid X receptor, thymine kinase promoter, and CAT(chloramphenicol acetyl transferase) were mixed with the plasmid DNA expressing retinoic acid receptor α , β, γ or retinoid X receptor α , β, γ to cotransfect skin cancer (HaCAT) cell line with lipofectamine (GibcoBRL). The cotransfected cell lines were cultured for 1 day on DMEM/10% FBS media, each retinol derivative (1µ M) was added and cultured for one more day, 5% CO₂, 37°C. The cells were washed with phosphate-buffered saline (PBS), and the proteins were isolated from each cells, and the activity of β -galactosidase and protein contents were measured to determine transfection efficacy, the transcription level of RAR or RXR receptor were measured with CAT ELISA (Roche Molecular Biochemical, Mannheim, Germany).

To determine the effect of retinol-(N-formyl aspartame) on retinoic acid receptors, each retinoic acid receptor (α , β, or γ) expression vector and activity measuring DR5-tk-CAT plasmid were introduced with liposomes. After treating cells with retinol-(N-formyl-aspartame), the cell extracts were isolated. The expression levels CAT were measured with a CAT ELISA to determine the effect of retinol-(N-formyl aspartame) on the activity of retinoic acid receptors.

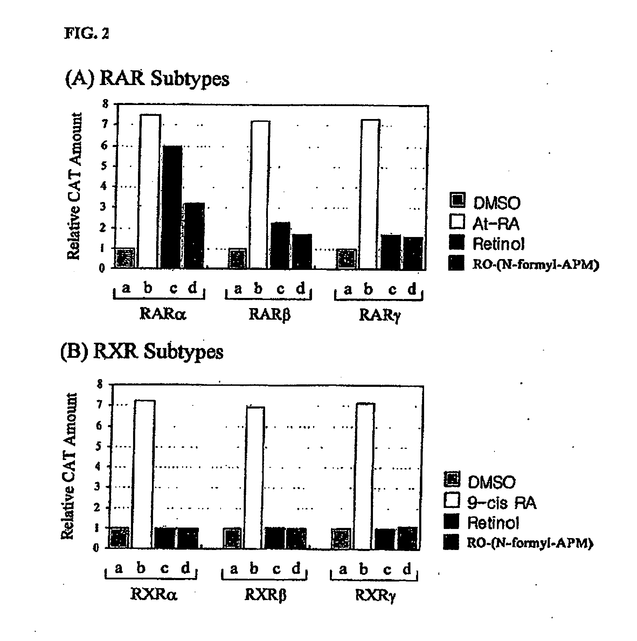

As shown in Figure 2, retinol-(N-formyl-aspartame) resulted in high activation of retinoic acid receptor a as retinol, and shows weak activation of retinoic acid receptor β , γ (A). Retinol and retinol-(N-formyl-aspartame) derivatives resulted in no activation of retinoid X receptor (α, β , γ) (B).

### Experimental Reference Example 3 : Activity test of retinoic acid receptor (RAR) treated with retinol-(mesaconic acid)

To analyze the effects of retinol-(mesaconic acid) on the activity of retinoic acid receptor, the Cos-1 cell line was utilized to measure the activity of retinoic acid receptor/ retinoid X receptor.

Recombinant DNA, DR5-tk-CAT consisting of DR5, effectors of retinoic acid receptor/ retinoid X receptor, thymine kinase promoter, and CAT (chloramphenicol acetyl transferase) were mixed with the plasmid DNA expressing retinoic acid receptor α , β , γ and utilized to cotransfect the skin cancer (HaCAT) cell line with lipofectamine (GibcoBRL). The cotransfected cell lines were cultured for 1 day in DMEM/10% FBS media, each retinol derivative (1µ M) was added and cultured for one more day, 5% CO₂ 37°C. The cells were washed with phosphate-buffered saline (PBS), and the proteins were isolated from each cells, and the activity of β galactosidase and protein contents were measured to determine transfection efficacy; the transcription revel of RAR receptor were measured with CAT ELISA.

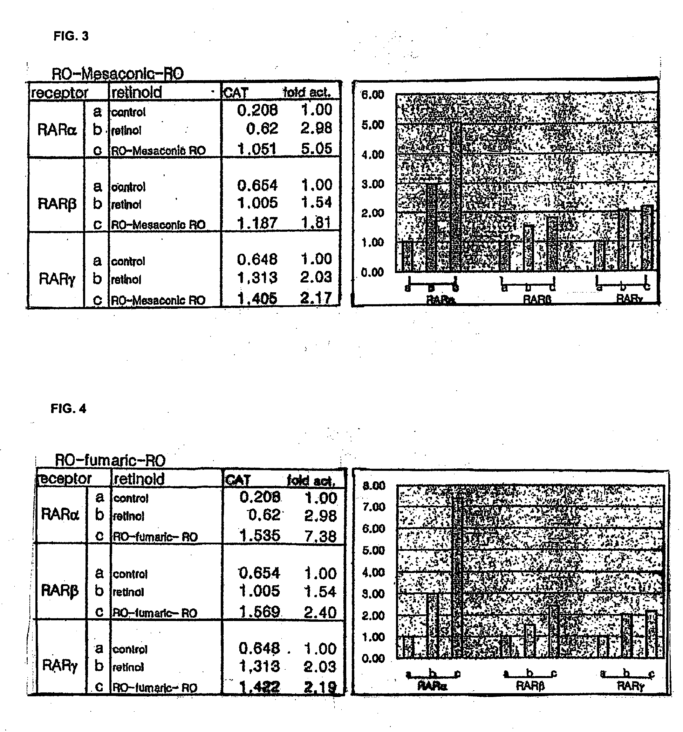

As shown in Figure 3, retinol-(mesaconic acid) resulted in high activation of retinoic acid receptor a as retinol, and resulted in weak activation of retinoic acid receptor β, γ in general.

### Experimental Reference Example 4 : Activity test of retinoic acid receptor (RAR) using retinol-(fumaric acid)

To analyze the effects of retinol-(fumaric acid) on the activity ofretinoic acid receptor, Cos-1 cell lines were utilized to measure the activity of retinoic acid receptor. All procedures were done as Experimental Example 3.

As shown in Figure 4, retinol-(fumaric acid) resulted in high activation of retinoic acid receptor a as retinol, and showed weak activation of retinoic acid receptor β , γ.

### Experimental Reference Example 5 : Activity test of retinoic acid receptor (RAR) using retinol-(retinol)

To analyze the effects of retinol-(retinol) on the activity of retinoic acid receptor, Cos-1 cell lines were utilized to measure the activity of retinoic acid receptor. All procedures were done as Experimental Example 3.

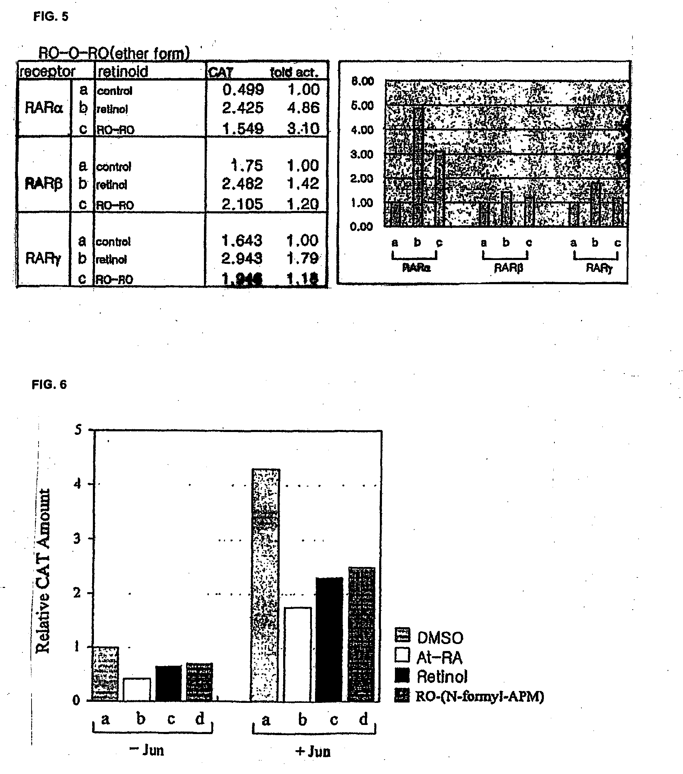

As shown in Figure 5, retinol-(retinol) resulted in high activation of retinoic acid receptor a as retinol, and showed weak activation of retinoic acid receptor β, γ.

### Experimental Example 6 : Activity inhibition test of activation protein-1 LAP-1) treated with retinol-(N-formyl aspartame)

Since the activation protein-1 (c-Jun is the constituent of Asp-1) is a transcription factor inducing expression of the collagenase enzyme, which is a major cause of skin wrinkle, the effect of wrinkle prevention was determined by the activity inhibition test of AP-1.

A CAT reporter (Coll-CAT) containing a collagen promoter including a AP-1 transcription factor binding site (TRE) was utilized to cotransfect the skin cancer (HaCAT) cell line. The effect of retinol derivatives on the activity of AP-1 was measured by CAT ELISA. In some cases, a vector expressing c-Jun or retinoic acid preceptors was utilized to cotransfect, and the effect of retinol derivatives on the transcription activity of c-Jun was analyzed.

With the assumption that the prepared retinol-(N-formyl-aspartame) derivatives which inhibit the activity of AP-1 causing skin wrinkle, would have the utility for cosmetics, the related investigations proceeded. Similar to the experiment of retinoic acid receptor, AP-1 (c-jun) expression vector and Coll-CAT plasmid for the activity measurement were introduced into the skin cancer (HaCAT) cell line with liposomes. After treating the cells with retinol-(N-formyl-aspartame) derivatives, the cell extracts were isolated. The expression levels of CAT were measured with a CAT ELISA to determine the effect of retinol-(N-formyl-aspartame) on the activity of the AP-1.

As shown in Figure 6, the expression of c-Jun elevated the expression of collagenase causing skin wrinkle, approximately 4.5 times, the treatment with retinol in the presence of retinoic acid receptor a lowered the expresson of collagenase by approximately 47%. The treatment of retinoic acid or the retinol-(N-formyl-aspartame lowered those approximately 60%, 44%, accordingly. In other cases, similar inhibition ratios were obtained (Figure 6, - c-Jun or + c-Jun). Therefore, those result showed that retinol-(N-formyl-aspartame) shows the similar inhibition effect on AP-1 activity as retinol, but less than retinoic acid.

### Experimental Reference Example 7 : Activity inhibition test of activation protein-1 (AP-1) treated with retinol-(mesaconic acid)

A CAT reporter (Colt-CAT) containing a collagen promoter including an AP-1 transcription factor binding site (TRE) was utilized to transfect the Cos-1 cell line. Activity inhibition tests of activation protein-1 (AP-1) similar to the experimental example 6 were performed.

When the expression level of collagenase in the presence of c-Jun expression was set as 100%, the treatment with retinol-(N-formyl-aspartame), retinoic acid, or retinol-(mesaconic acid) lowered the expression of collagenase approximately 42%, 50%, or 30%, respectively. Therefore, those result showed that retinol-(mesaconic acid) has less effect on AP-1 activity inhibition than retinoic acid or retinol.

### Experimental Reference Example 8 : Activity inhibition test of activation protein-1 : AP-1 using retinol-(fumaric acid)

A CAT reporter (Coll-CAT) containing a collagen promoter including an AP-1 transcription factor binding site (TRE) was utilized to transfect the Cos-1 cell line. Activity inhibition tests of activation protein-1 (AP-1) similar to the experimental example 6 were performed

When the expression level of collagenase in the presence of c-Jun expression was set as 100%, the treatment with retinol-(fumaric acid) or retinoic acid lowered the expression of collagenase approximately 42%, 50% respectively. Therefore, those result showed that retinol-(fumaric acid) has no significant effect on AP-1 activity inhibition than retinoic acid or retinol.

### Experimental Reference Example 9 : Activity inhibition test of activation protein AP-1 using retinol-(retinol)

A CAT reporter (Coll-CAT) containing a collagen promoter including an AP-1 transcription factor binding site (TRE) was utilized to transfect the Cos-1 cell line. Activity inhibition tests of activation protein-1 (AP-1) similar to the experimental example 6 were performed

When the expression level of collagenase in the presence of the c-Jun expression was set as 100%, the treatment with retinol, retinoic acid or retinol-(retinol) lowered the expression of collagenase approximately 43%, 47.5%, 41.5% respectively. Therefore, those result showed that retinol-(retinol) has a similar effect on AP-1 activity inhibition compared to retinoic acid or retinol.

### INDUSTRIAL UTILITY

The retinol derivatives of this invention showed better stability against light than the retinol of the prior art, which was known to be maintained without any alteration even after 10 days.

The retinol derivatives of this invention resulted in the high activation of retinoic acid receptor a as retinol, the weak activation of retinoic acid receptor β γ in general and no activation of retinoid X receptor (α, β, γ).

Also, in the case of inhibition of activation protein-1, the retinol derivatives of this invention resulted in similar inhibition of c-jun activity as retinol, similar to the effect on retinoic acid receptor α.

Therefore, the retinol derivatives of this invention can be used effectively as a medication, cosmetic, soap, shampoo, or functional food for the purpose of prevention and improvement of skin ageing caused by wrinkle, roughness, dryness, or abnormal keratinization.

## Claims

1. A retinol derivative comprising a carboester linkage between retinol and a peptide having a functional group of COOH, wherein the retinol derivative has the formula I 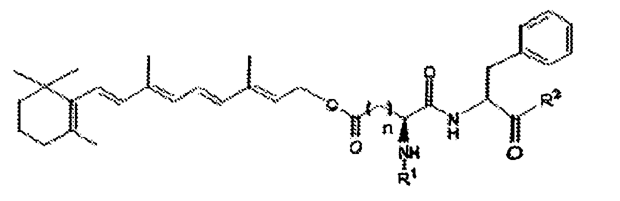 wherein either R¹ is H, CHO, retinoic acid (RA), Ac or Boc;
R² is OH, OCH₃, OC₂H₅, retinol, or conjugated linoleic acid (CLA); and
n is an integer of 1-6,
or n is 1, R² is OCH₃, R¹ is 3,3-dimethyl-butyl or an N-protecting-group, and the phenylalanine is L-phenylalanine.

2. The retinol derivative according to claim 1, wherein either n is 1, R¹ is H, R² is OCH₃ and the phenylalanine is L-phenylalanine or n is 1, R¹ is 3,3-dimethyl-butyl, R² is OCH₃ and the phenylalanine is L-phenylalanine.

3. A method for preparing retinol carboester derivatives according to claim 1 consisting of the following stages:
1) a converting stage of retinyl acetate to pure retinol by reacting retinyl acetate with an inorganic salt in methanolic solvent and extracting retinol with ether;
2) a binding stage of a peptide having a functional group of COOH to retinol under the presence of a condensing agent and a catalyzing agent in the solvent; and
3) a separating, purifying and recovering stage of said retinol carboester derivatives.

4. A method according to claim 3, wherein said converting stage 1) is **characterized in that** the extracting solvent is an ether such as diethylether or tetrahydrofuran (THF).

5. A method according to claim 3 or claim 4, wherein said binding stage is **characterized in that** the condensing agent is selected from the group comprising N-N'-dicyclohexylcarbodiimide (DCC), 1-ethyl-3-(3-dimethylaminopropyl) carbodiimide hydrochloride (EDCI), N,N'-carbonyldiimidazole (CDI), N,N'-sulfuryldiimidazole (SDI), and SO₂Cl.

6. A pharmaceutical composition for the treatment or the prevention of skin diseases comprising retinol derivatives according to claim 1 or claim 2.

7. The pharmaceutical composition of claim 6, wherein the skin disease is selected from the group consisting of skin cancer, pimple, wrinkle with age, pigmentation, coarse skin, and flappy skin.

8. The pharmaceutical composition according to claim 6 or claim 7, wherein the administration of said pharmaceutical composition is selected from the group consisting of oral administration, local administration, transdermal administration, intranasal administration, inhalation, ocular administration, rectal administration, intravenous administration, intraperitoneal administration, intramuscular administration, intraarterial administration or subcutaneous administration.

9. The pharmaceutical composition according to claim 8, wherein the transdermal administration is achieved via a pharmaceutical formulation selected from the group consisting of a lotion, an ointment, a gel, a cream, a patch and a spray.

10. A cosmetic composition for the prevention of skin abnormality comprising retinol derivatives according to claim 1 or claim 2.

11. The cosmetic composition according to claim 10, wherein the skin abnormality is selected from the group consisting of freckles and senile chromelasma.

12. A cosmetic composition according to claim 10 or claim 11, wherein said cosmetic composition is selected from the group consisting of a toning lotion, nourishing lotion, massage cream, nourishing cream, pack, gel and skin adhesive.

13. Soap and shampoo compositions for the prevention of skin abnormality comprising a retinol derivative according to claim 1 or claim 2.

14. A food composition for the prevention of skin abnormality comprising retinol derivatives according to claim 1 or claim 2.

15. The food composition according to claim 14, wherein the skin abnormality is selected from the group consisting of pimple, freckles, wrinkle, pigmentation, coarse skin and flappy skin.

## Patentansprüche

1. Retinol-Derivat, umfassend eine Carbonester-Bindung zwischen Retinol und einem Peptid mit einer funktionellen COOH-Gruppe, wobei das Retinol-Derivat die Formel I hat 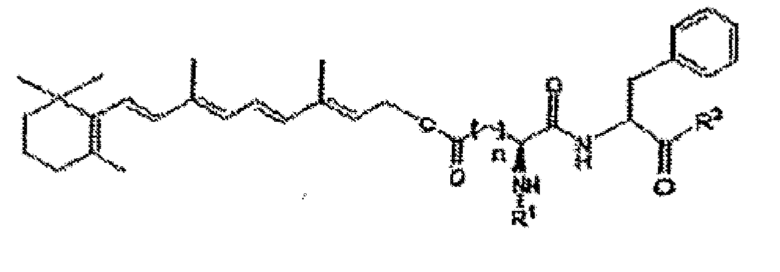 wobei R¹ entweder H, CHO, Retinolsäure (RA), Ac oder Boc ist;
R² ist OH, OCH₃, OC₂H₅, Retinol, oder konjugierte Linolsäure (CLA); und n eine ganze Zahl zwischen 1-6,
oder n 1 ist, R² OCH₃ ist, R¹ 3,3-Dimethyl-Butyl oder eine N-geschützte Gruppe ist und Phenylalanin L-Phenylalanin ist.

2. Retinol-Derivat nach Anspruch 1, wobei entweder n 1 ist, R¹ H ist, R² OCH₃ ist, und Phenylalanin L Phenylalanin ist, oder n 1 ist, R¹ 3,3-Dimethyl-Butyl ist, R² OCH₃ ist, und Phenylalanin L-Phenylalanin ist

3. Verfahren zur Herstellung des Retinol-Carbonester-Derivats nach Anspruch 1, bestehend aus den folgenden Stufen:
1) Umwandeln von Retinyl-Acetat zu reinem Retinol durch Reagieren von Retinyl-Acetat mit einem anorganischen Salz in einem methanolischen Lösungsmittel und Extrahieren des Retinols mit Ether,
2) Binden eines Peptids mit einer funktionellen COOH-Gruppe an Retinol in Anwesenheit eines kondensierenden Stoffs und einem katalysierenden Stoff in einem Lösungsmittel; und
3) Trennen, Reinigen- Ernten des Retinol-Carbonester-Derivats.

4. Verfahren nach Anspruch 3, wobei das Umwandeln 1) **dadurch** charakterisiert wird, dass das Extraktionslösungsmittel ein Ether, wie z.B. Diethylether oder Tetrahydrofuran (THF) ist.

5. Verfahren nach Anspruch 3 oder 4, wobei das Binden **dadurch** charakterisiert ist, dass der kondensierende Wirkstoff ausgewählt ist aus der Gruppe, bestehend aus N-N'-Dicyclohexylcarbodümid ( D C C), 1-Ethyl-3-(3-Dimethylaminopropyl)carbodümid-Hydrochlorid (EDCI), N,N'-Carbonyldiimidazol (CDI), N,N'-Sulfuryldiimidazol (SDI), und SO₂Cl.

6. Pharmazeutische Zusammensetzung für die Behandlung oder die Vorbeugung von Hautkrankheiten, umfassend die Retinol-Derivate nach Anspruch 1 oder 2.

7. Pharmazeutische Zusammensetzung nach Anspruch 6, wobei die Hautkrankheit ausgewählt ist aus der Gruppe bestehend aus Hautkrebs, Pickel, Altersfalten, Pigmentierung, rauer Haut und schlaffer Haut.

8. Pharmazeutische Zusammensetzung nach Anspruch 6 oder 7, wobei die Verabreichung der pharmazeutischer Zusammensetzung ausgewählt ist aus der Gruppe bestehend aus oraler Verabreichung, lokaler Verabreichung, transdermaler Verabreichung, intranasaler Verabreichung, Inhalation, okularer Verabreichung, rektaler Verabreichung, intravenöser Verabreichung, intraperitonealer Verabreichung, intramuskulärer Verabreichung, intraarterieller Verabreichung und subkutaner Verabreichung.

9. Pharmazeutische Zusammensetzung nach Anspruch 8, wobei die transdermale Verabreichung erreicht wird durch eine pharmazeutische Formulierung, ausgewählt aus der Gruppe bestehend aus einer Lotion, eine Salbe, einem Gel, einer Creme, einem Pflaster und einem Spray.

10. Kosmetische Zusammensetzung für die Vorbeugung von Hautanomalie umfassend die Retinol-Derivate nach Anspruch 1 oder 2.

11. Kosmetische Zusammensetzung nach Anspruch 10, wobei die Hautanomalie ausgewählt ist aus der Gruppe bestehend aus Sommersprossen und Chromelasma senile.

12. Kosmetische Zusammensetzung nach Anspruch 10 oder 11, wobei die kosmetische Zusammensetzung ausgewählt ist aus der Gruppe bestehend aus einer Tönungslotion, Nährbalsam, Massagecreme, Nährcreme, Packung, einem Gel und einem Hautpflaster.

13. Seifen- und Shampoo-Zusammensetzungen zur Vorbeugung von Haut-Anomalie, umfassend ein Retinol-Derivat nach Anspruch 1 oder 2.

14. Nahrungszusammensetzung zur Vorbeugung von Haut-Anomalie, umfassend Retinol-Derivate nach Anspruch 1 oder 2.

15. Nahrungszusammensetzung nach Anspruch 14, wobei die Haut-Anomalie ausgewählt ist aus der Gruppe bestehend aus einem Pickel, Sommersprossen, Falten, Pigmentierung, rauer Haut und schlaffer Haut.

## Revendications

1. Un dérivé de rétinol comprenant une liaison de type carboester entre le rétinol et un peptide possédant un groupe fonctionnel COOH, dans lequel le dérivé de rétinol est de la formule 1 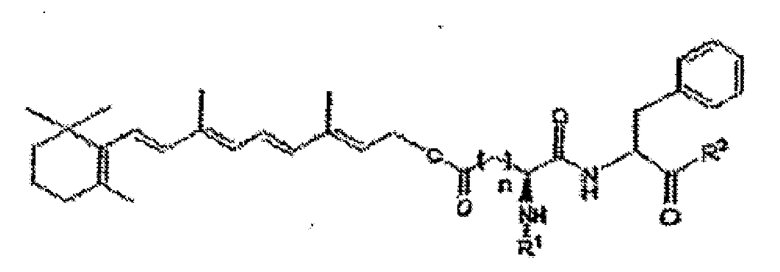 dans laquelle R¹ est H, CHO, l'acide rétinoïque (RA), Ac ou Boc;
R² est OH, OCH₃, OC₂H₅, le rétinol, ou l'acide linoléique conjugué (CLA); et n est un nombre entier de 1-6,
ou n est 1, R² est OCH₃, R¹ est 3,3-diméthyle-butyle ou un groupe de protection de N, et la phénylalanine est la L-phénylalanine.

2. Le dérivé de rétinol selon la revendication 1, dans lequel n est 1, R¹ est H, R² est OCH₃ et la phénylalanine est la L-phénylalanine, ou n est 1, R¹ est 3,3-diméthyle-butyle, R² est OCH₃ et la phénylalanine est la L-phénylalanine.

3. Une méthode de préparation d'un dérivé de rétinol de type carboester selon la revendication 1 qui consiste en les étapes suivantes:
1) une étape de conversion de l'acétate du rétinol à rétinol pur dans laquelle l'acétate du rétinol réagit avec un sel inorganique dans un solvant du type méthanol et le rétinol est extrait avec un éther;
2) une étape de relier un peptide qui a un groupe fonctionnel COOH à rétinol dans la présence d'un agent de condensation et un agent catalytique dans un solvant; et
3) séparer, purifier et obtenir le-dit dérivé du retinol du type carboester .

4. Une méthode selon la revendication 3, dans laquelle l'étape de de conversion 1) est **caractérisée par** le solvant d'extraction soyant un éther tel que l'éther diéthylique ou tetrahydrofuran(THF).

5. Une méthode selon la revendication 3 ou 4, dans laquelle l'étape de relier est **caractérisée par le fait que** l'agent de condensation est sélectionné d' un groupe qui consiste en N-N'-dicyclohexylcarbodiimide (DCC), 1-éthyl-3-(3-diméthylaminopropyle) carbodiimide hydrochloride (EDC1), N,N'-carbonyldiimidazole (CD1), N,N'-sulfurylediimidazole(SD1), et SO₂Cl.

6. Une composition pharmaceutique pour le traitement ou la prévention des maladies de la peau comprenant des dérivés du retinol selon la revendication 1 ou 2.

7. La composition pharmaceutique selon la revendication 6, dans laquelle la maladie de la peau est sélectionnée d'un groupe qui consiste en le cancer de la peau, des boutons, des rides de l'âge, la pigmentation, la peau bourrue, et la peau flasque.

8. La composition pharmaceutique selon la revendication 6 ou 7, dans laquelle l'administration de la-dite composition pharmaceutique est sélectionné d'un groupe qui consiste en l'administration orale, l'administration locale, l'administration transdermique, l'administration intranasale, l'inhalation, l'administration occulaire, l'administration rectale, l'administration intraveineuse, l'administration intrapéritoneale, l'administration intramusculaire, l'administration intraarterielle et l'administration sous-cutanée.

9. La composition pharmaceutique selon la revendication 8, dans laquelle l'administration transdermique est effectuée par une formulation pharmaceutique sélectionnée du groupe qui consiste en une lotion, un onguent, un gel, une crème, un pansement et un spray.

10. Une composition cosmétique pour la prévention d'une anomalie de la peau qui comprend des dérivés de rétinol selon la revendication 1 ou 2.

11. La composition cosmétique selon la revendication 10, dans laquelle l'anomalie de la peau est sélectionnée d'un groupe qui consiste en des taches de rousseur et chromelasma sénile.

12. Une composition cosmétique selon la revendication 10 ou 11, dans laquelle ladite composition cosmétique est sélectionnée du groupe qui consiste en une lotion de coloration, un baume nourrissant, une crème de massage, une crème nourrissante, une masque (pack), un gel et un pansement.

13. Savon et compositions du shampooing pour la prévention d'une anomalie de la peau qui comprend un dérivé de rétinol selon la revendication 1 ou 2.

14. Une composition de nourriture pour la prévention d'une anomalie de la peau qui comprend des dérivés du retinol selon la revendication 1 ou 2

15. La composition de nourriture selon la revendication 14, dans laquelle l'anomalie de la peau est sélectionnée d'un groupe qui consiste en des boutons, des taches de rousseur, des rides, la pigmentation, la peau bourrue et la peau flasque.
